(19) Europäisches Patentamt
European Patent Office
Office européen des brevets

(11) **EP 4 516 255 A1**

(12) **EUROPEAN PATENT APPLICATION**
published in accordance with Art. 153(4) EPC

(43) Date of publication:
05.03.2025 Bulletin 2025/10

(51) International Patent Classification (IPC):
**A61B 34/35** (2016.01) **B25J 3/00** (2006.01)

(21) Application number: 23831381.1

(22) Date of filing: 26.06.2023

(52) Cooperative Patent Classification (CPC):
**A61B 34/35; B25J 3/00; B25J 13/08**

(86) International application number:
**PCT/JP2023/023580**

(87) International publication number:
**WO 2024/004941 (04.01.2024 Gazette 2024/01)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: 27.06.2022 JP 2022102885

(71) Applicant: KAWASAKI JUKOGYO KABUSHIKI KAISHA
Kobe-shi, Hyogo 650-8670 (JP)

(72) Inventors:
• **TANABE, Masataka**
  JP)
• **TAKANO, Yusuke**
  JP)

(74) Representative: **Hoffmann Eitle Patent- und Rechtsanwälte PartmbB**
**Arabellastraße 30**
**81925 München (DE)**

(54) **SURGERY SUPPORT SYSTEM AND SURGERY SUPPORT SYSTEM CONTROL METHOD**

(57) In a robotic surgical system (100), an operation apparatus (2) includes a display (24) to rotate so as to be tilted with respect to a horizontal plane, and a tilt detection sensor (29a) to detect a tilt of the display (24) with respect to the horizontal plane. The control device (130) is configured or programmed to correct translational movement of a predetermined surgical instrument (4) based on the tilt detected by the tilt detection sensor (29a).

*FIG.19*

## Description

Technical Field

The present disclosure relates to a robotic surgical system and a control method for a robotic surgical system.

Background Art

Conventionally, a robotic surgical system disclosed in U.S. Patent Application Publication No. 2012/0283876 is known. The robotic surgical system disclosed in U.S. Patent Application Publication No. 2012/0283876 includes a master controller and a slaved robotic manipulator. The master controller includes a display and an operation unit. A surgical instrument is attached to the slaved robotic manipulator. An image of a surgical site captured by an endoscope is displayed on the display of the master controller. The surgical instrument attached to the slaved robotic manipulator is moved by an operator operating the operation unit of the master controller while viewing the image of the surgical site displayed on the display. In the robotic surgical system disclosed in U.S. Patent Application Publication No. 2012/0283876, the display angle of the display of the master controller can be adjusted.

Prior Art

Patent Document

Patent Document 1: U.S. Patent Application Publication No. 2012/0283876

Summary of the Invention

As described in U.S. Patent Application Publication No. 2012/0283876, when the display angle, which is the tilt angle of the display of the master controller with respect to a horizontal plane, is changed, a direction in which the endoscope captures an image and the direction of the line of sight of the operator viewing the display change as compared with those before the display angle of the display is changed. The inventors of the present disclosure have found that a direction in which the operator operates the operation unit changes due to a change in the direction of the line of sight of the operator viewing the display. In other words, the inventors of the present disclosure have found that when the operator operates the operation unit to translationally move the surgical instrument along a desired direction, the direction in which the operator operates the operation unit changes before and after the display angle of the display is changed, even though the desired direction in which the operator wants to translationally move the surgical instrument does not change. Therefore, when the display angle of the display is changed, the surgical instrument cannot be accurately translationally moved in the desired direction intended by the operator.

The present disclosure is intended to solve the above problem. The present disclosure aims to provide a robotic surgical system and a control method for a robotic surgical system each capable of accurately translationally moving a surgical instrument in a desired direction intended by an operator even when the tilt of a display changes.

A robotic surgical system according to a first aspect of the present disclosure includes a surgical apparatus including a first robot arm having an endoscope attached to a distal end thereof, and a second robot arm having a predetermined surgical instrument other than the endoscope attached to a distal end thereof, an operation apparatus including an operation unit to receive an operation for the predetermined surgical instrument or the endoscope, and a control device configured or programmed to perform a control to move the predetermined surgical instrument or the endoscope based on a received operation. The operation apparatus includes a display to display an image captured by the endoscope and rotate so as to be tilted with respect to a horizontal plane, and a tilt detection sensor to detect a tilt of the display with respect to the horizontal plane. The control device is configured or programmed to correct translational movement of the predetermined surgical instrument based on the tilt detected by the tilt detection sensor.

In the robotic surgical system according to the first aspect of the present disclosure, as described above, the control device is configured or programmed to correct the translational movement of the predetermined surgical instrument based on the tilt detected by the tilt detection sensor to detect the tilt of the display with respect to the horizontal plane. Accordingly, even when the tilt of the display with respect to the horizontal plane changes, the translational movement of the predetermined surgical instrument is corrected by the control device. Therefore, even when the tilt of the display changes, the surgical instrument can be accurately translationally moved in the desired direction intended by an operator. Furthermore, the tilt of the display with respect to the horizontal plane can be relatively easily detected by the tilt detection sensor. Therefore, even when the tilt of the display changes, the surgical instrument can be accurately translationally moved in the desired direction intended by the operator while the tilt of the display with respect to the horizontal plane is easily detected.

A control method for a robotic surgical system according to a second aspect of the present disclosure is a control method for a robotic surgical system including a surgical apparatus including a first robot arm having an endoscope attached to a distal end thereof and a second robot arm having a predetermined surgical instrument other than the endoscope attached to a distal end thereof, an operation apparatus including an operation unit to receive an operation for the predetermined surgical instrument or the endoscope, and a control device configured or programmed to perform a control to move the predetermined surgical instrument or the endoscope based on a received operation, and the control method includes detecting a tilt with respect to a horizontal plane of a display operable to display an image captured by the endoscope, the display being operable to rotate so as to be tilted with respect to the horizontal plane, and correcting translational movement of the predetermined surgical instrument based on a detected tilt.

As described above, the control method for the robotic surgical system according to the second aspect of the present disclosure includes detecting, using a tilt detection sensor, the tilt with respect to the horizontal plane of the display operable to display the image captured by the endoscope, the display being operable to rotate so as to be tilted with respect to the horizontal plane, and correcting the translational movement of the predetermined surgical instrument based on the detected tilt. Accordingly, even when the tilt of the display with respect to the horizontal plane changes, the translational movement of the predetermined surgical instrument is corrected. Therefore, it is possible to provide the control method for the robotic surgical system capable of accurately translationally moving the surgical instrument in the desired direction intended by an operator even when the tilt of the display changes. Furthermore, the tilt of the display with respect to the horizontal plane can be relatively easily detected by the tilt detection sensor. Therefore, it is possible to provide the control method for the robotic surgical system capable of accurately translationally moving the surgical instrument in the desired direction intended by the operator while easily detecting the tilt of the display with respect to the horizontal plane even when the tilt of the display changes.

According to the present disclosure, the surgical instrument can be accurately translationally moved in the desired direction intended by the operator even when the tilt of the display changes.

Brief Description of the Drawings

FIG. 1 is a diagram showing the configuration of a robotic surgical system according to an embodiment.
FIG. 2 is a diagram showing the configuration of a robot arm according to the embodiment.
FIG. 3 is a diagram showing a pair of forceps.
FIG. 4 is a perspective view showing the configuration of an arm operation unit according to the embodiment.
FIG. 5 is a diagram for illustrating translational movement of the robot arm.
FIG. 6 is a diagram for illustrating rotational movement of the robot arm.
FIG. 7 is a diagram showing an operation unit according to the embodiment.

FIG. 8 is a diagram showing the configuration of a right-handed operation unit according to the embodiment.
FIG. 9 is a diagram showing the configuration of a left-handed operation unit according to the embodiment.
FIG. 10 is a diagram showing the configuration of foot pedals according to the embodiment.
FIG. 11 is a side view of a remote control apparatus according to the embodiment.
FIG. 12 is a diagram showing a state in which a monitor is tilted.
FIG. 13 is a control block diagram of the robotic surgical system according to the embodiment.
FIG. 14 is a control block diagram of the robot arm according to the embodiment.
FIG. 15 is a control block diagram of the remote control apparatus according to the embodiment.
FIG. 16 is a diagram showing an operator and an HC coordinate system.
FIG. 17 is a diagram showing a surgical instrument, an endoscope, and an endoscope coordinate system.
FIG. 18 is a diagram for illustrating operations performed when the operation unit receives an operation.
FIG. 19 is a flowchart of a control method for the robotic surgical system according to the embodiment.
FIG. 20 is a side view of a remote control apparatus according to a modified example.

Modes for Carrying Out the Invention

Configuration of Robotic Surgical System

The configuration of a robotic surgical system 100 according to this embodiment is now described. The robotic surgical system 100 includes a surgical robot 1 and a remote control apparatus 2. The surgical robot 1 and the remote control apparatus 2 are examples of a surgical apparatus and an operation apparatus, respectively.

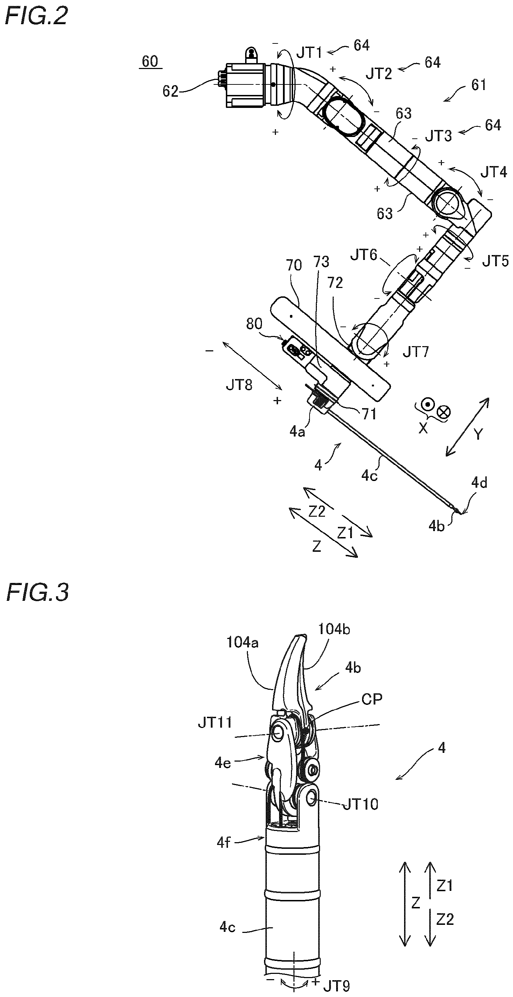

In this specification, the longitudinal direction of a shaft 4c of a surgical instrument 4 is defined as a Z direction, as shown in FIG. 2. The distal end side of the surgical instrument 4 is defined as a Z1 side, and the proximal end side of the surgical instrument 4 is defined as a Z2 side. A direction perpendicular to the Z direction is defined as an X direction. A direction perpendicular to the Z direction and the X direction is defined as a Y direction.

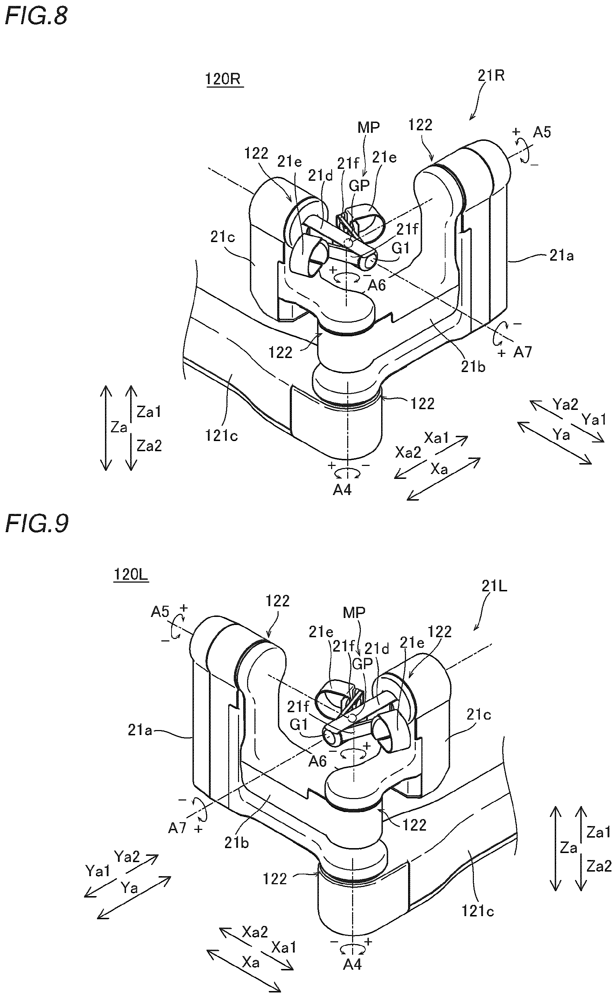

In this specification, as shown in FIG. 8, a direction along a vertical direction is defined as a Za direction. In the Za direction, a first side is defined as a Za1 side, and a second side is defined as a Za2 side. A direction perpendicular to the Z direction is defined as an Xa direction. In the Xa direction, a first side is defined as an Xa1 side, and a second side is defined as an Xa2 side.

A direction perpendicular to the Za direction and the Xa direction is defined as a Ya direction. In the Ya direction, a first side is defined as a Ya1 side, and a second side is defined as a Ya2 side. The Xa direction and the Ya direction are directions along a horizontal plane.

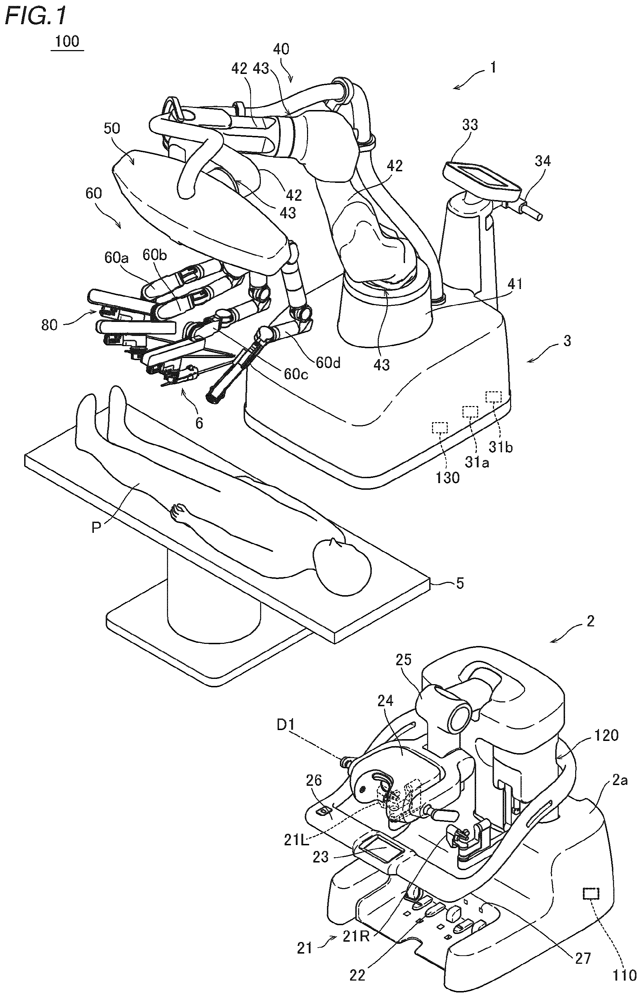

As shown in FIG. **1,** the surgical robot 1 is arranged in an operating room. The remote control apparatus 2 is spaced apart from the surgical robot **1.** An operator such as a doctor inputs a command to the remote control apparatus 2 to cause the surgical robot 1 to perform a desired operation. The remote control apparatus 2 transmits the input command to the surgical robot **1.** The surgical robot 1 operates based on the received command. The surgical robot 1 is arranged in the operating room that is a sterilized sterile field.

Configuration of Surgical Robot

As shown in FIG. 1, the surgical robot 1 includes a medical cart 3, a positioner 40, an arm base 50, a plurality of robot arms 60, and arm operation units 80.

The medical cart 3 moves the positioner 40. The medical cart 3 includes an input 33. The input 33 receives operations to move the positioner 40, the arm base 50, and the plurality of robot arms 60 or change their postures mainly in order to prepare for surgery before the surgery. The medical cart 3 includes an operation handle 34 to receive an operator's steering operation.

The positioner 40 includes a 7-axis articulated robot, for example. The positioner 40 is arranged on the medical cart 3. The positioner 40 adjusts the position of the arm base 50. The positioner 40 moves the position of the arm base 50 three-dimensionally.

The positioner 40 includes a base 41 and a plurality of links 42 coupled to the base 41. The plurality of links 42 are connected to each other by joints 43.

The arm base 50 is attached to a distal end of the positioner 40. A proximal end of each of the plurality of robot arms 60 is attached to the arm base 50. Each of the plurality of robot arms 60 is able to take a folded and stored posture. The arm base 50 and the plurality of robot arms 60 are covered with sterile drapes and used. Moreover, each of the robot arms 60 supports the surgical instrument 4.

The plurality of robot arms 60 are arranged. Specifically, four robot arms 60a, 60b, 60c, and 60d are arranged. The robot arms 60a, 60b, 60c, and 60d have the same or similar configurations as each other. The robot arms 60a, 60b, and 60d are examples of a second robot arm, and the robot arm 60c is an example of a first robot arm.

As shown in FIG. 2, each robot arm 60 includes an arm portion 61, a first link 72, a second link 73, and a translation mechanism 70. The robot arm 60 includes a JT1 axis, a JT2 axis, a JT3 axis, a JT4 axis, a JT5 axis, a JT6 axis, and a JT7 axis as rotation axes and a J8 axis as a linear motion axis. The JT1 axis, the JT2 axis, the JT3 axis, the JT4 axis, the JT5 axis, the JT6 axis, and the JT7 axis are rotation axes of joints 64 of the arm portion 61. Furthermore, the JT7 axis is a rotation axis of the first link 72. The JT8 axis is a linear motion axis along which the translation mechanism 70 moves the second link 73 relative to the first link 72 along the Z direction.

The arm portion 61 includes a 7-axis articulated robot arm. The first link 72 is arranged at a distal end of the arm portion 61. An arm operation unit 80 is attached to the second link 73. The translation mechanism 70 is arranged between the first link 72 and the second link 73. A holder 71 that holds the surgical instrument 4 is arranged on the second link 73.

The surgical instrument 4 is attached to a distal end of each of the plurality of robot arms 60. The surgical instrument 4 includes a replaceable instrument, or an endoscope 6 to capture an image of a surgical site, for example. The surgical instrument 4 as the instrument includes a driven unit 4a, a pair of forceps 4b, and the shaft 4c that connects the driven unit 4a to the pair of forceps 4b. The driven unit 4a, the shaft 4c, and the pair of forceps 4b are arranged along the Z direction.

As shown in FIG. 1, the endoscope 6 is attached to the distal end of one of the plurality of robot arms 60, such as the robot arm 60c, and surgical instruments 4 other than the endoscope 6 are attached to the distal ends of the remaining robot arms 60a, 60b, and 60d, for example. The endoscope 6 is attached to one of two robot arms 60b and 60c arranged in the center among the four robot arms 60 arranged adjacent to each other.

Configuration of Instrument

As shown in FIG. 3, the pair of forceps 4b is arranged at a distal end of the instrument, for example. At the distal end of the instrument, in addition to the pair of forceps 4b, a pair of scissors, a grasper, a needle holder, a microdissector, a stable applier, a tacker, a suction cleaning tool, a snare wire, a clip applier, etc. are arranged as instruments having joints. At the distal end of the instrument, a cutting blade, a cautery probe, a washer, a catheter, a suction orifice, etc. are arranged as instruments having no joint.

The pair of forceps 4b includes a first support 4e that supports the proximal end sides of jaw members 104a and 104b on the distal end side such that the proximal end sides of the jaw members 104a and 104b are rotatable about a JT11 axis, and a second support 4f that supports the proximal end side of the first support 4e on the distal end side such that the proximal end side of the first support 4e is rotatable about a JT10 axis. The shaft 4c rotates about a JT9 axis. The jaw members 104a and 104b pivot about the JT11 axis to open and close. A portion of the first support 4e on the Z1 direction side, that is the distal end side, has a U-shape.

As shown in FIG. 2, the arm operation unit 80 is attached to the robot arm 60. Specifically, the arm operation unit 80 is attached to the second link 73.

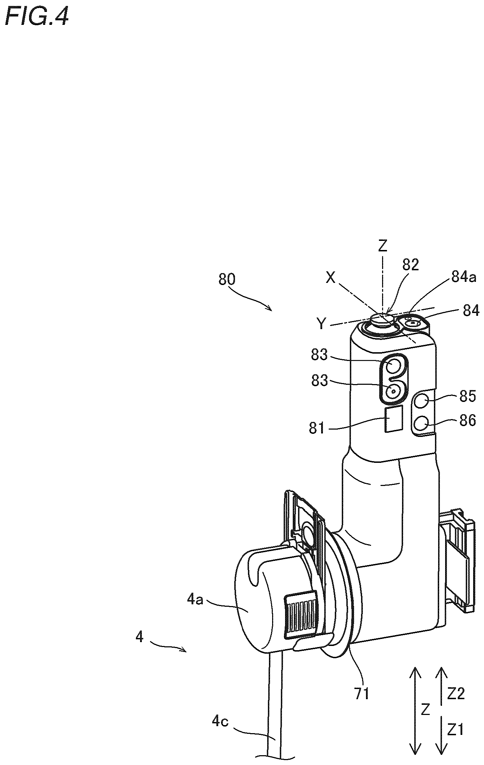

As shown in FIG. 4, the arm operation unit 80 includes an enable switch 81, a joystick 82, and linear switches 83, a mode switching button 84, a mode indicator 84a, a pivot button 85, and an adjustment button 86.

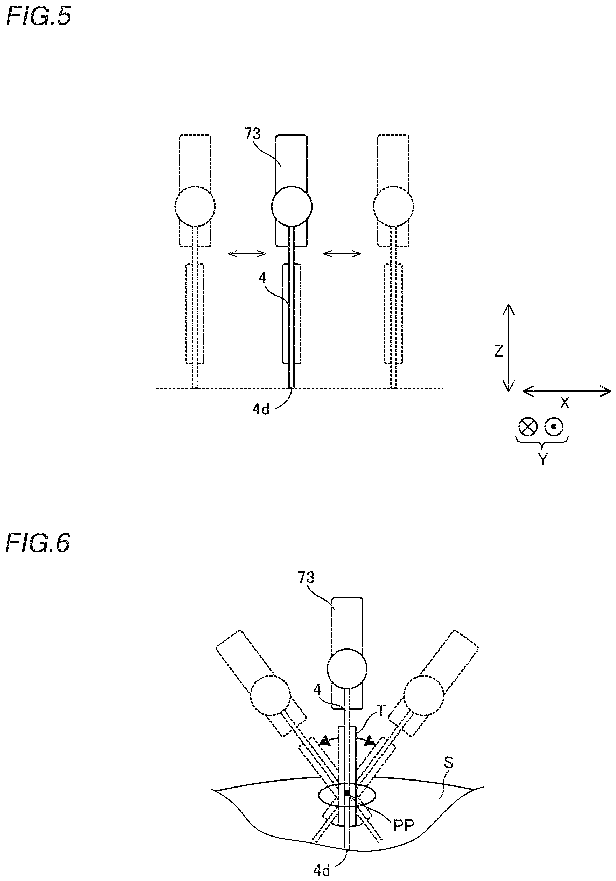

The enable switch 81 enables or disables movement of the robot arm 60 in response to the joystick 82 and the linear switches 83. The joystick 82 is an operation tool to control movement of the surgical instrument 4 by the robot arm 60. The linear switches 83 are switches to move the surgical instrument 4 in a direction along the longitudinal direction of the surgical instrument 4. The mode switching button 84 is a button to switch between a mode for translationally moving the surgical instrument 4 as shown in FIG. 5 and a mode for rotationally moving the surgical instrument 4 as shown in FIG. 6. The mode indicator 84a indicates a switched mode. The pivot button 85 is a button to teach the pivot position PP that serves as a fulcrum for movement of the surgical instrument 4 attached to the robot arm 60. The adjustment button 86 is a button to optimize the position of the robot arm 60.

Remote Control Apparatus

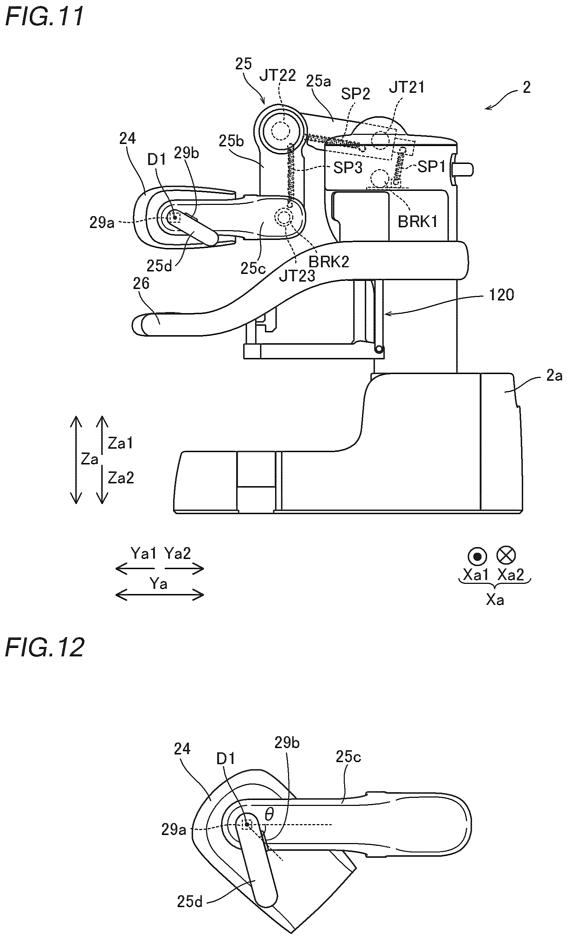

As shown in FIG. 1, the remote control apparatus 2 is arranged inside or outside the operating room, for example. The remote control apparatus 2 includes a main body 2a, operation units 120, foot pedals 22, a touch panel 23, a monitor 24, a support arm 25, a support bar 26, foot detectors 27, an angle sensor 29a shown in FIG. 11, and switches 29b shown in FIG. 11. The monitor 24 is an example of a display. The angle sensor 29a is an example of a tilt detection sensor.

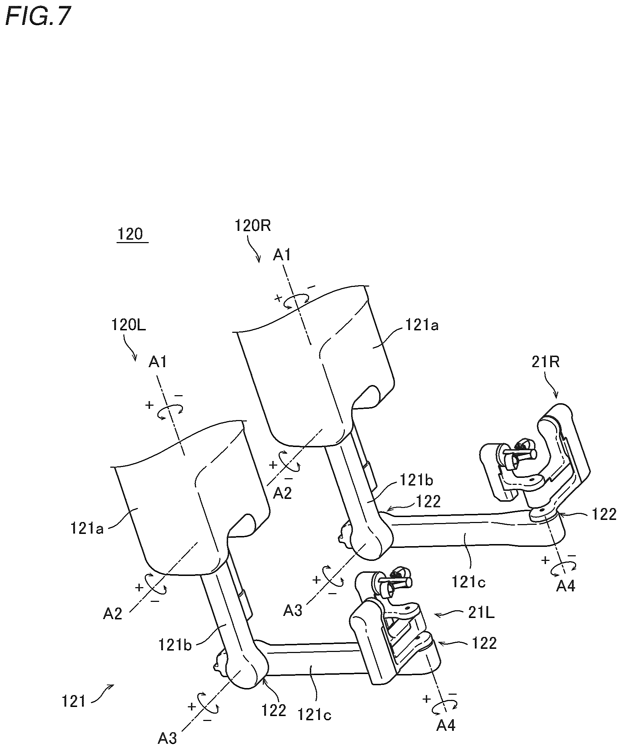

As shown in FIG. 1, the operation units 120 receive operations for the endoscope 6 or the surgical instruments 4 other than the endoscope 6. The operation units 120 are supported by the main body 2a. As shown in FIG. 7, the operation units 120 includes a left-handed operation unit 120L that is located on the left side as viewed from the operator such as a doctor and is to be operated by the left hand of the operator, and a right-handed operation unit 120R that is located on the right side and is to be operated by the right hand of the operator. The left-handed operation unit 120L and the right-handed operation unit 120R have the same or similar configurations as each other.

The operation units 120 include substantially L-shaped arms 121 and operation handles 21. The arms 121 each include a link 121a, a link 121b, and a link 121c. The upper end side of the link 121a is attached to the main body 2a such that the link 121a is rotatable about an A1 axis along the vertical direction. The upper end side of the link 121b is attached to the lower end side of the link 121a such that the link 121b is rotatable about an A2 axis along a horizontal direction. A first end side of the link 121c is attached to the lower end side of the link 121b such that the link 121c is rotatable about an A3 axis along the horizontal direction. The operation handle 21 is attached to a second end side of the link 121c such that the operation handle 21 is rotatable about an A4 axis. The links are connected to each other by joints 122.

The arm 121 supports the operation handle 21. The arm 121 supports the operation handle 21 such that the operation handle 21 is movable within a predetermined three-dimensional operation range. Specifically, the arm 121 supports the operation handle 21 such that the operation handle 21 is movable in an upward-downward direction, a right-left direction, and a forward-rearward direction. The robot arm 60 is moved three-dimensionally so as to correspond to a three-dimensional operation on the arm 121.

The operation handles 21 include an operation handle 21R shown in FIG. 8 to be operated by the right hand of the operator, and an operation handle 21L shown in FIG. 9 to be operated by the left hand of the operator. FIG. 8 shows the reference position of the right-handed operation unit 120R, and FIG. 9 shows the reference position of the left-handed operation unit 120L. The operation handle 21R and the operation handle 21L have the same or similar configurations as each other. The operation handles 21 each include a link 21a, a link 21b, a link 21c, and a link 21d operated by the operator such as a doctor. The link 21a rotates about the A4 axis. The link 21b is attached to the link 21a so as to be rotatable about an A5 axis. The link 21c is attached to the link 21b so as to be rotatable about an A6 axis. The link 21d is attached to the link 21c so as to be rotatable about an A7 axis. The links are connected to each other by joints 122. Each of the links 21a, 21b, and 21c has an L-shape.

The operation handles 21 each includes a pair of grip members 21f to be opened and closed by the operator. The grip members 21f each include an elongated plate-shaped lever member, and proximal ends of the pair of grip members 21f are rotatably connected to a proximal end G1 of the link 21d. Cylindrical finger insertion portions 21e are provided on the grip members 21f. The operator inserts his/her right fingers into a pair of finger insertion portions 21e to operate the operation handle 21R. The operator inserts his/her left fingers into a pair of finger insertion portions 21e to operate the operation handle 21L. The proximal ends of the pair of grip members 21f are connected to the link 21d, and an angle between the pair of grip members 21f is increased or decreased such that an opening angle between the jaw member 104a and the jaw member 104b is changed. A magnet is provided on one of the grip members 21f, and a Hall sensor is provided on the link 21d. When the operator opens and closes the grip members 21f, the magnet and the Hall sensor function as an angle detection sensor, and the Hall sensor outputs the opening angle. As the angle detection sensor, the Hall sensor may be provided on the grip member 21f, and the magnet may be provided on the link 21d. Alternatively, the magnet or the Hall sensor may be provided as the angle detection sensor on both the grip members 21f.

The intersection of a plurality of rotation axes of the operation unit 120 is called a gimbal point GP. Specifically, the gimbal point GP is a point at which the A4 axis, the A5 axis, the A6 axis, and the A7 axis intersect with each other. The gimbal point GP is located in the link 21d to which the pair of grip members 21f are attached. The gimbal point GP exists individually in each of the left-handed operation unit 120L and the right-handed operation unit 120R.

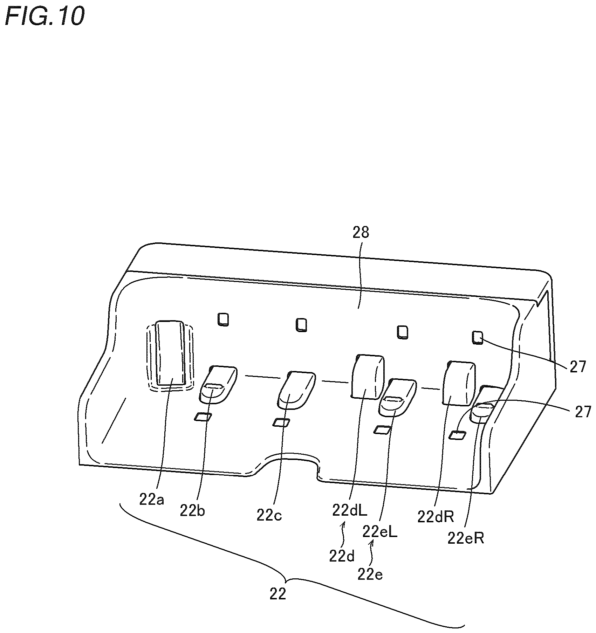

As shown in FIG. 10, a plurality of foot pedals 22 are provided to perform functions related to the surgical instruments 4. The plurality of foot pedals 22 are arranged on a base 28. The foot pedals 22 include a switching pedal 22a, a clutch pedal 22b, a camera pedal 22c, an incision pedal 22d, and a coagulation pedal 22e. The switching pedal 22a, the clutch pedal 22b, the camera pedal 22c, the incision pedal 22d, and the coagulation pedal 22e are operated by the foot of the operator. The incision pedal 22d includes an incision pedal 22dR for a right robot arm 60, and an incision pedal 22dL for a left robot arm 60. The coagulation pedal 22e includes a coagulation pedal 22eR for the right robot arm 60 and a coagulation pedal 22eL for the left robot arm 60.

The switching pedal 22a switches the robot arms 60 to be operated by the operation handles 21. The clutch pedal 22b performs a clutch operation to temporarily disconnect an operation connection between the robot arms 60 and the operation handles 21. While the clutch pedal 22b is being pressed by the operator, operations by the operation handles 21 are not transmitted to the robot arms 60. While the camera pedal 22c is being pressed by the operator, the operation handle 21 can operate a robot arm 60 to which the endoscope 3 is attached. While the incision pedal 22d or the coagulation pedal 22e is being pressed by the operator, an electrosurgical device is activated.

The foot detectors 27 detect the foot of the operator that operates the foot pedals 22. The foot detectors 27 detect the foot that hovers above their corresponding foot pedals 22. The foot detectors 27 are arranged on the base 28.

As shown in FIG. 11, the monitor 24 is a scope-type display that displays an image captured by the endoscope 6. As shown in FIG. 12, the monitor 24 rotates so as to be tilted with respect to the horizontal plane. Specifically, the monitor 24 rotates about a D1 axis along the Xa direction. The D1 axis is an example of a first axis.

As shown in FIG. 11, the support arm 25 supports the monitor 24 so as to align the height of the monitor 24 with the height of the face of the operator such as a doctor. The support arm 25 includes a first link 25a, a second link 25b, a third link 25c, and grips 25d. A first end of the first link 25a is attached to the main body 2a. A joint JT21 is disposed at the first end of the first link 25a. A second end of the first link 25a is connected to a first end of the second link 25b by a joint JT22. A second end of the second link 25b is connected to a first end of the third link 25c by a joint JT23. The monitor 24 is rotatably attached to the third link 25c. The grips 25d are arranged at the third link 25c. The grips 25d are arranged on both the Xa1 side and the Xa2 side of the third link 25c. The first link 25a and the second link 25b are examples of a link. The third link 25c is an example of a link or a holder.

A spring SP1 is arranged at a proximal end of the first link 25a. The first link 25a is lifted by the spring SP1. A spring SP2 is arranged at the first link 25a. The second link 25b is lifted by the spring SP2. A spring SP3 is arranged at the second link 25b. The third link 25c is lifted by the spring SP3. A brake BRK1 is arranged on the proximal end side of the first link 25a. The brake BRK1 fixes the joint JT21 such that the joint JT21 does not rotate. A brake BRK2 is arranged at the joint JT23 by which the second link 25b is connected to the third link 25c. The brake BRK2 fixes the joint JT23 such that the joint JT23 does not rotate. The springs SP1, SP2, and SP3 are arranged to support the weights of the support arm 25 and the monitor 24. The brakes BRK1 and BRK2 are electromagnetic brakes that are activated without excitation and prevent the joints JT21 and JT23 from moving even when an external force is applied thereto. Only one or two of the springs SP1, SP2, and SP3 may be provided. Furthermore, only the brake BRK1 or the brake BRK2 may be provided. Alternatively, a brake may be provided at the joint JT22.

In this embodiment, the switches 29b switch between a state in which a change in the posture of the support arm 25 is permitted and a state in which the change in the posture is not permitted. The switches 29b are arranged on the grips 25d. When the operator presses the switches 29b, the brakes BRK1 and BRK2 are released, and the operator can change the posture of the support arm 25 by gripping and moving the grips 25d. The operator can change the tilt angle of the monitor 24 with respect to the horizontal plane by gripping and tilting the grips 25d. A brake BRK3 may be provided to fix rotation of the monitor 24 and the third link 25c. When the operator presses the switches 29b, the brake BRK3 may be released such that the tilt angle of the monitor 24 with respect to the horizontal plane can be changed in addition to changing the posture of the support arm 25.

In this embodiment, the angle sensor 29a detects the tilt of the monitor 24 with respect to the horizontal plane. The monitor 24 rotates about the D1 axis with respect to the third link 25c. The angle sensor 29a detects the rotation angle $\theta$ of the monitor 24 about the D1 axis along the horizontal plane. The angle sensor 29a detects the rotation angle $\theta$ of the monitor 24 with respect to the third link 25c. The angle sensor 29a is an encoder that detects the rotation angle $\theta$ of the monitor 24, for example.

As shown in FIG. 1, the touch panel 23 is arranged on the support bar 26. When a sensor provided in the vicinity of the monitor 24 detects the head of the operator, the operator is enabled to operate the surgical robot 1 using the remote control apparatus 2. The operator operates the operation units 120 and the foot pedals 22 while visually recognizing an affected area on the monitor 24. Thus, a command is input to the remote control apparatus 2. The command input to the remote control apparatus 2 is transmitted to the surgical robot 1. The support arm 25 is an example of a support.

Configuration of Control System

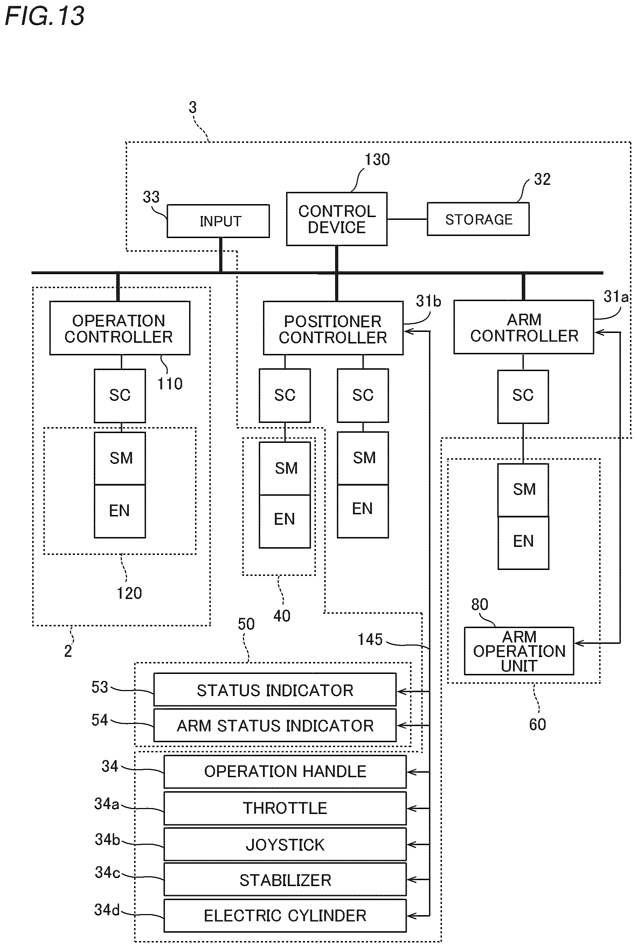

As shown in FIG. 13, the robotic surgical system 100 includes a control device 130, an arm controller 31a, a positioner controller 31b, and operation controllers 110.

The control device 130 is accommodated in the medical cart 3 to communicate with the arm controller 31a and the positioner controller 31b, and controls the entire robotic surgical system 100. Specifically, the control device 130 communicates with and controls the arm controller 31a, the positioner controller 31b, and the operation controllers 110. The control device 130 is connected to the arm controller 31a, the positioner controller 31b, and the operation controllers 110 through a LAN, for example. The control device 130 is arranged inside the medical cart 3.

The arm controller 31a is arranged for each of the plurality of robot arms 60. That is, the same number of arm controllers 31a as the plurality of robot arms 60 are placed inside the medical cart 3.

As shown in FIG. 13, the input 33 is connected to the control device 130 through a LAN, for example. A status indicator 53, an arm status indicator 54, the operation handle 34, a throttle 34a, a joystick 33b, a stabilizer 34c, and an electric cylinder 34d are connected to the positioner controller 31b via a wire line 145 by means of a communication network that allows information to be shared with each other by using serial communication. Although FIG. 13 shows that the status indicator 53, the arm status indicator 54, etc. are all connected to one wire line 145, in reality, the wire line 145 is arranged for each of the status indicator 53, the arm status indicator 54, the operation handle 34, the throttle 34a, the joystick 33b, the stabilizer 34c, and the electric cylinder 34d.

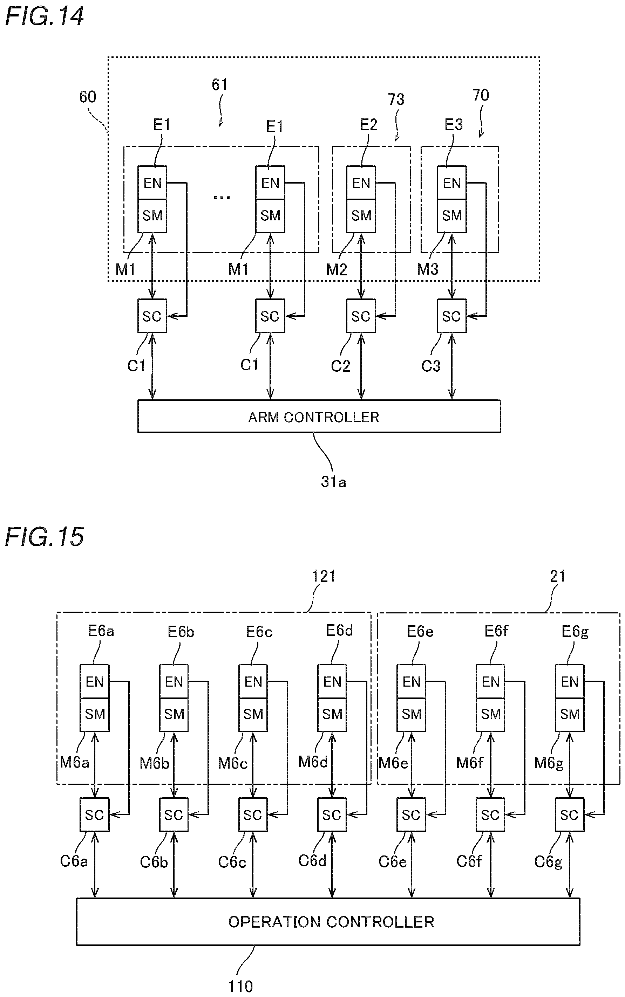

As shown in FIG. 14, the arm portion 61 includes a plurality of servomotors M1, encoders E1, and speed reducers so as to correspond to a plurality of joints 64. The encoders E1 detect rotation angles of the servomotors M1. The speed reducers slow down rotation of the servomotors M1 to increase the torques. Inside the medical cart 3, servo controllers C1 that control the servomotors M1 are arranged adjacent to the arm controller 31a. In addition, the encoders E1 that detect the rotation angles of the servomotors M1 are electrically connected to the servo controllers C1.

Servomotors M2 that rotate driven members provided in the driven unit 4a of the surgical instrument 4, encoders E2, and speed reducers are arranged in the second link 73. The encoders E2 detect rotation angles of the servomotors M2. The speed reducers slow down rotation of the servomotors M2 to increase the torques. In the medical cart 3, servo controllers C2 are provided to control the servomotors M2 to drive the surgical instrument 4. The encoders E2 that detect the rotation angles of the servomotors M2 are electrically connected to the servo controllers C2. A plurality of servomotors M2, a plurality of encoders E2, and a plurality of servo controllers C2 are arranged.

The translation mechanism 70 includes a servomotor M3 to translationally move the surgical instrument 4, an encoder E3, and a speed reducer. The encoder E3 detects a rotation angle of the servomotor M3. The speed reducer slows down rotation of the servomotor M3 to increase the torque. In the medical cart 3, a servo controller C3 is provided to control the servomotor M3 to translationally move the surgical instrument 4. The encoder E3 that detects the rotation angle of the servomotor M3 is electrically connected to the servo controller C3.

As shown in FIG. 13, the positioner controller 31b is arranged in the medical cart 3. The positioner controller 31b controls the positioner 40 and the medical cart 3. Servomotors SM, encoders EN, speed reducers, and servo controllers SC are provided in the positioner 40 so as to correspond to a plurality of joints 43 of the positioner 40. Servomotors SM that drive a plurality of front wheels of the medical cart 3, encoders EN, speed reducers, servo controllers SC, and brakes are provided in the medical cart 3.

As shown in FIG. 15, servomotors M6a, M6b, M6c, M6d, M6e, M6f, and M6g are provided in the operation units 120 so as to correspond to the A1 axis, the A2 axis, the A3 axis, the A4 axis, the A5 axis, the A6 axis, and the A7 axis, which are rotation axes, respectively. Servo controllers C6a, C6b, C6c, C6d, C6e, C6f, and C6g are provided in the medical cart 3 to control the servomotors. Encoders E6a, E6b, E6c, E6d, E6e, E6f, and E6g that detect rotation angles of the servomotors are electrically connected to the servo controllers, respectively. The servomotors, the servo controllers, and the encoders are provided in each of the left-handed operation unit 120L and the right-handed operation unit 120R.

The control device 130 controls the servomotors via the operation controllers 110 to generate torques that cancel gravitational torques generated on the rotation axes of the servomotors according to the postures of the operation units 120. Thus, the operator can operate the operation units 120 with a relatively small force.

The control device 130 generates torques on the rotation axes of the servomotors according to operations on the operation units 120 via the operation controllers 110, and controls the servomotors to assist the operation of the operator. Thus, the operator can operate the operation units 120 with a relatively small force.

Correction of Translational Movement of Surgical Instrument

The control device 130 performs a control to move the endoscope 6 or the surgical instrument 4 other than the endoscope 6 based on operations received by the operation units 120. Specifically, as shown in FIGS. 8 and 9, the control device 130 sets an operation unit reference point MP for each of the operation units 120. The operation unit reference point MP is also called a mapping point. The operation unit reference point MP is set at the gimbal point GP, for example. The control device 130 sets a surgical instrument reference point CP shown in FIG. 3 for the surgical instrument 4. When the operation unit 120 is operated, the control device 130 performs a control to move the surgical instrument 4 such that the surgical instrument reference point CP moves in response to movement of the operation unit reference point MP. The operation of moving the surgical instrument 4 such that the surgical instrument reference point CP moves in response to movement of the operation unit reference point MP is called following. As shown in FIG. 3, the surgical instrument reference point CP is set at the center of a Z1 direction side portion of the first support 4e in a JT11 axis direction. The surgical instrument reference point CP is called a tool center point or a clevis point.

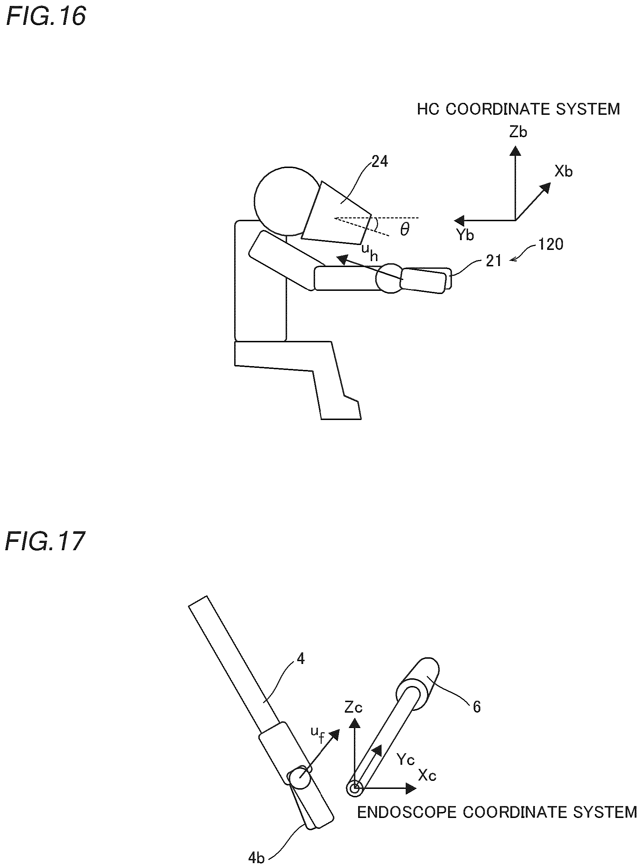

As shown in FIG. 16, a coordinate system of the operation unit 120 is set in the operation unit 120. Hereinafter, the coordinate system of the operation unit 120 is referred to as an HC coordinate system. As shown in FIG. 17, a coordinate system of the endoscope 6 is set in the endoscope 6. Hereinafter, the coordinate system of the endoscope 6 is referred to as an endoscope coordinate system. For example, when the operator moves the operation unit 120 along the Yb direction of the HC coordinate system, the surgical instrument 4 moves along the Yc direction of the endoscope coordinate system, which is a direction in which the endoscope 6 extends.

The inventors of the present disclosure have found that a direction in which the operator operates the operation unit 120 changes due to a change in the direction of the line of sight of the operator viewing the monitor 24. For example, assume that the operator attempts to translationally move the surgical instrument 4 along the Yc direction of the endoscope coordinate system when the monitor 24 is tilted obliquely downward. At this time, it has been found that the operator moves the operation unit 120 obliquely downward intersecting with the Yb direction as shown by a translation vector uh in FIG. 16, instead of the Yb direction of the HC coordinate system. Therefore, the surgical instrument 4 is translationally moved in a direction different from a direction intended by the operator. Note that uh in FIG. 16 is a translation vector that indicates a direction in which the operation unit 120 is moved as viewed from the HC coordinate system.

Therefore, in this embodiment, the control device 130 corrects translational movement of the surgical instrument 4 based on the tilt detected by the angle sensor 29a. The surgical instrument 4 is an instrument with the pair of forceps 4b arranged at the distal end, for example. Specifically, when a translational movement operation for the surgical instrument 4 is received, the control device 130 corrects a direction of the translational movement of the surgical instrument 4 based on the tilt detected by the angle sensor 29a such that the surgical instrument 4 translationally moves along the direction in which the endoscope 6 extends. That is, even when the translation vector uh of the operation unit 120 is directed obliquely upward, the control device 130 corrects a translation vector uf such that the translation vector uf follows the Yc direction of the endoscope coordinate system. Note that uf is a translation vector that indicates the movement direction of the pair of forceps 4b as viewed from a robot reference coordinate system.

When the monitor 24 is arranged along the horizontal plane, the control device 130 does not correct the direction of the translational movement of the surgical instrument 4 when an operation is received to translationally move the surgical instrument 4 along the forward-rearward direction as viewed by the operator. That is, when the monitor 24 is arranged along the horizontal plane, the control device 130 translationally moves the surgical instrument 4 such that the surgical instrument 4 translationally moves along the Yc direction of the endoscope coordinate system when an operation is received to translationally move the surgical instrument 4 along the Yb direction of the HC coordinate system.

In this embodiment, as shown in the following equation (1), when a translational movement operation for the surgical instrument 4 is received, the control device 130 corrects the direction of the translational movement of the surgical instrument 4 based on a posture term that represents the posture of the endoscope 6 based on the coordinate system of the endoscope 6 and a correction term that corrects the posture term based on the tilt detected by the angle sensor 29a. Specifically, when a translational movement operation for the surgical instrument 4 is received, the control device 310 corrects the translation vector uf of the surgical instrument 1 by multiplying the translation vector uh of the surgical instrument 1 based on the coordinate system of the operation unit 120 by a posture matrix Hcam of the endoscope 3 based on the coordinate system of the endoscope 3 and a posture correction matrix Rx based on the tilt detected by the angle sensor 29a.

$$\text{uf} = \text{Hcam} \cdot \text{Rx} \cdot \text{uh} \ \dots \ (1)$$

Hcam represents a posture matrix in a camera coordinate system as viewed from the robot reference coordinate system. Rx represents a posture correction matrix according to the rotation angle $\theta$ of the monitor 24 about the D1 axis. Rx varies according to the rotation angle $\theta$. Furthermore, Rx corresponds to the tilt of the monitor 24 with respect to the horizontal plane. That is, the control device 130 corrects the translational movement of the surgical instrument 1 based on the rotation angle $\theta$ about the D1 axis detected by the angle sensor 29a.

In this embodiment, the control device 130 corrects the translational movement of the surgical instrument 4 based on the tilt detected by the angle sensor 29a during surgery in which an operation for the surgical instrument 4 is received by the remote control apparatus 2. Specifically, the control device 130 corrects the translational movement of the surgical instrument 4 based on the tilt detected by the angle sensor 29a while the following, which is the operation of moving the surgical instrument 4, is being performed. When the operator presses the switches 29b during surgery to change the tilt angle of the monitor 24 with respect to the horizontal plane, Rx in the above equation (1) is changed so as to correspond to the changed tilt angle of the monitor 24. Then, the translational movement of the surgical instrument 4 is corrected based on the changed Rx.

Method for Calculating Operation Amount

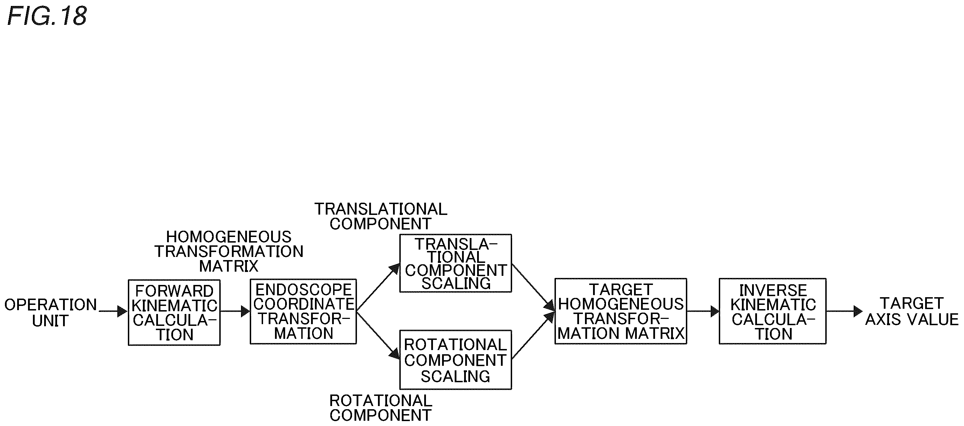

A method for calculating an input value of the operation unit 120 when an operation of the operator is received is now described. As shown in FIG. 18, the operation of the operator is received by the operation unit 120. Thus, the axis values of the A1 axis, the A2 axis, the A3 axis, the A4 axis, the A5 axis, the A6 axis, and the A7 axis of the operation unit 120 are input to the operation controller 110. The control device 130 performs a forward kinematic calculation based on each axis value input to the operation controller 110. Thus, a homogeneous transformation matrix that represents the displacement and rotation of the operation unit reference point MP from the reference posture is updated as an input value from the operation unit 120. The forward kinematic calculation is means for calculating, from the axis values of a link mechanism, the displacement and rotation of a focused portion of the mechanism. The control device 130 converts the homogeneous transformation matrix that represents the input from the operation unit 120 according to the viewing direction of the endoscope **6.** At this time, the translational movement of the surgical instrument 4 is corrected based on the above equation (1). The obtained homogeneous transformation matrix includes a translational component for the translational movement of the surgical instrument 4 and a rotational component for the rotation of the surgical instrument 4, which generate a movement corresponding to the input of the operation unit 120 within the field of view of the endoscope 6. The control device 130 performs scaling on the translational component and the rotational component. The term "scaling" refers to multiplying the translational component and the rotational component by a scaling value, which is a ratio between an operation amount received by the operation unit 120 and the actual movement amount of the surgical instrument 4. The control device 130 calculates a target homogeneous transformation matrix based on the scaled translational component and rotational component. The control device 130 performs an inverse kinematic calculation on the target homogeneous transformation matrix. The control device 130 calculates each axis value that is the target of the robot arm 60 and the surgical instrument 4 by the inverse kinematic calculation.

Control method for Robotic Surgical System

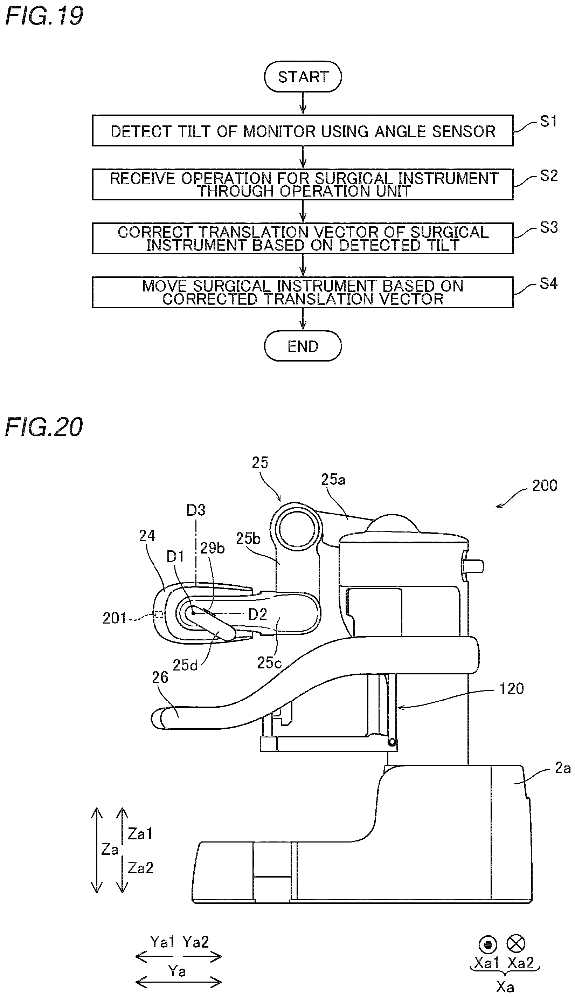

A control method for the robotic surgical system 100 is now described.
As shown in FIG. 19, in step S1, the angle sensor 29a detects the tilt of the monitor 24 with respect to the horizontal plane.
In step S2, the operation unit 120 receives an operation for the surgical instrument 4.
In step S3, the control device 130 corrects the translation vector of the surgical instrument 4 using the above equation (1) based on the detected tilt.
In step S4, the control device 130 performs a control to move the surgical instrument 4 based on the corrected translation vector.

Advantages of This Embodiment

The control device 130 is configured or programmed to correct translational movement of the surgical instrument 4 based on the tilt detected by the angle sensor 29a to detect the tilt of the monitor 24 with respect to the horizontal plane. Accordingly, even when the tilt of the monitor 24 with respect to the horizontal plane changes, the translational movement of the surgical instrument 4 is corrected by the control device 130. Therefore, even when the tilt of the monitor 24 changes, the surgical instrument 4 can be accurately translationally moved in the desired direction intended by the operator. Furthermore, the tilt of the monitor 24 with respect to the horizontal plane can be relatively easily detected by the angle sensor 29a. Therefore, even when the tilt of the monitor 24 changes, the surgical instrument 4 can be accurately translationally moved in the desired direction intended by the operator while the tilt of the monitor 24 with respect to the horizontal plane is easily detected.
The control device 130 is configured or programmed to correct the direction of the translational movement of the surgical instrument 4 based on the tilt detected by the angle sensor 29a such that the surgical instrument 4 translationally moves along the direction in which the endoscope 6 extends when a translational movement operation for the surgical instrument 4 is received. Accordingly, even when the tilt of the monitor 24 changes, the surgical instrument 4 can be accurately translationally moved along the direction in which the endoscope 6 extends.
The control device 130 is configured or programmed to correct the direction of the translational movement of the surgical instrument 4 based on the posture term that represents the posture of the endoscope 6 based on the coordinate system of the endoscope 6 and the correction term that corrects the posture term based on the tilt detected by the angle sensor 29a when a translational movement operation for the surgical instrument 4 is received. Accordingly, the posture term that represents the posture of the endoscope 6 is corrected by the correction term based on the tilt detected by the angle sensor 29a, and thus the surgical instrument 4 can be appropriately translationally moved to follow the posture of the endoscope 6.

The control device 130 is configured or programmed to correct the translation vector uf of the surgical instrument 4 by multiplying the translation vector uh of the surgical instrument 4 based on the coordinate system of the operation unit 120 by the posture matrix Hcam of the endoscope 6 based on the coordinate system of the endoscope 6 and the posture correction matrix Rx based on the tilt detected by the angle sensor 29a when a translational movement operation for the surgical instrument 4 is received. Accordingly, the translation vector uf of the surgical instrument 4 is corrected by the posture matrix Hcam of the endoscope 6 and the posture correction matrix Rx, and thus the surgical instrument 4 can be appropriately translationally moved to follow the posture of the endoscope 6.

The remote control apparatus 2 includes the support arm 25 to support the monitor 24. The support arm 25 includes the first link 25a, the second link 25b, the third link 25c, the joints JT21, JT22, and JT23, the brake BRK1 to fix the joint JT21 such that the joint JT21 does not rotate, the brake BRK2 to fix the joint JT23 such that the joint JT23 does not rotate, the spring SP1 to lift the first link 25a, the spring SP2 to lift the second link 25b, and the spring SP3 to lift the third link 25c. Accordingly, even when an external force is applied to the support arm 25, the joints JT21 and JT23 can be fixed by the brakes BRK1 and BRK2 so as not to move. Furthermore, the weights of the support arm 25 and the monitor 24 are supported by the springs SP1, SP2, and SP3, and thus the posture of the support arm 25 is maintained.

The control device 130 is configured or programmed to correct the translational movement of the surgical instrument 4 based on the tilt detected by the angle sensor 29a during surgery in which an operation for the surgical instrument 4 is received by the remote control apparatus 2. Accordingly, even when the tilt of the monitor 24 is changed during surgery, the surgical instrument 4 can be translationally moved in the desired direction intended by the operator.

The angle sensor 29a is operable to detect the rotation angle θ of the monitor 24 about the D1 axis along the horizontal plane, and the control device 130 is configured or programmed to correct the translational movement of the surgical instrument 4 based on the rotation angle θ about the D1 axis detected by the angle sensor 29a. Accordingly, the tilt of the monitor 24 with respect to the horizontal plane can be easily detected based on the rotation angle θ of the monitor 24 about the D1 axis.

The remote control apparatus 2 includes the switches 29b to switch between a state in which a change in the tilt angle of the monitor 24 with respect to the horizontal plane is permitted and a state in which the change in the tilt angle is not permitted. Accordingly, a change in the tilt of the monitor 24 at the timing unintended by the operator can be reduced or prevented by the switches 29b.

Modified Examples

The embodiment disclosed this time must be considered as illustrative in all points and not restrictive. The scope of the present disclosure is not shown by the above description of the embodiment but by the scope of claims for patent, and all modifications or modified examples within the meaning and scope equivalent to the scope of claims for patent are further included.

While the example in which the direction of translational movement of the surgical instrument 4 is corrected such that the surgical instrument 4 translationally moves along the Yc direction of the endoscope coordinate system in which the endoscope 6 extends has been shown in the aforementioned embodiment, the present disclosure is not limited to this. For example, the direction of translational movement of the surgical instrument 4 may be corrected such that the surgical instrument 4 translationally moves along a direction other than the Yc direction of the endoscope coordinate system.

While the example in which the translation vector is corrected based on the above equation (1) has been shown in the aforementioned embodiment, the present disclosure is not limited to this. In the present disclosure, the translation vector may be corrected based on an equation other than the above equation (1).

While the example in which the translational movement of the surgical instrument 4 is corrected when an operation for the surgical instrument 4 is received by the remote control apparatus 2 has been shown in the aforementioned embodiment, the present disclosure is not limited to this. For example, based on an operation of the operator, a state in which the translational movement of the surgical instrument 4 is corrected and a state in which the translational movement of the surgical instrument 4 is not corrected may be switched.

While the example in which the control device 130 corrects the translational movement of the surgical instrument 4 based on the rotation angle θ of the monitor 24 about the D1 axis detected by the angle sensor 29a has been shown in the aforementioned embodiment, the present disclosure is not limited to this. For example, an acceleration sensor 201 may be arranged as in a remote control apparatus 200 according to a modified example shown in FIG. 20. The acceleration sensor 201 is arranged at a monitor 24 on the Ya1 direction end side of a third link 25c of the remote control apparatus 200. The acceleration sensor 201 detects at least the tilt of the monitor 24 with respect to a horizontal plane. Specifically, the acceleration sensor 201 detects the rotation angles of the monitor 24 about a D1 axis along the horizontal plane, a D2 axis along the horizontal plane and perpendicular to the D1 axis, and a D3 axis along a vertical direction. Thus, the tilt of the monitor 24 can be easily detected by the acceleration sensor 201. Furthermore, the acceleration sensor 201 can easily detect a three-dimensional change in the tilt of the monitor 24. A control device 130 corrects translational movement of a surgical instrument 4 based on the rotation angles about the D1 axis, the D2 axis, and the D3 axis detected by the acceleration sensor 201. Accordingly, even when the tilt of the monitor 24 is changed three-dimensionally, the surgical instrument 4 can be accurately translationally moved in a desired direction intended by an operator. Specifically, the translational movement of the surgical instrument 4 is corrected based on the following equation (2).

$$uf = Hcam \cdot Mcomp \cdot uh \; ... \; (2)$$

Mcomp represents a posture correction matrix that is changed according to the rotation angles of the monitor 24 about the D1, D2, and D3 axes. The acceleration sensor 201 is an example of a tilt detection sensor. The D2 axis and the D3 axis are examples of a second axis and a third axis, respectively.

While the example in which the switches 29b switch between a state in which a change in the tilt angle of the monitor 24 with respect to the horizontal plane is permitted and a state in which the change in the tilt angle is not permitted has been shown in the aforementioned embodiment, the present disclosure is not limited to this. For example, the switches 29b may not be provided, and the tilt angle of the monitor 24 may be freely changed by the operator.

While the example in which four robot arms 60 are provided has been shown in the aforementioned embodiment, the present disclosure is not limited to this. In the present disclosure, the number of robot arms 60 may be any number as long as at least one robot arm 60 is provided.

While the example in which each of the arm portion 61 and the positioner 40 includes a 7-axis articulated robot has been shown in the aforementioned embodiment, the present disclosure is not limited to this. For example, each of the arm portion 61 and the positioner 40 may include an articulated robot having an axis configuration other than the 7-axis articulated robot. The axis configuration other than the 7-axis articulated robot includes six axes or eight axes, for example.

While the example in which the surgical robot 1 includes the medical cart 3, the positioner 40, and the arm base 50 has been shown in the aforementioned embodiment, the present disclosure is not limited to this. For example, the surgical robot 1 may not include the medical cart 3, the positioner 40, or the arm base 50, but may include only the robot arms 60.

The functionality of the elements disclosed herein may be implemented using circuitry or processing circuitry that includes general purpose processors, special purpose processors, integrated circuits, application specific integrated circuits (ASICs), conventional circuitry and/or combinations thereof that are configured or programmed to perform the disclosed functionality. Processors are considered processing circuitry or circuitry as they include transistors and other circuitry therein. In the present disclosure, the circuitry, units, or means are hardware that carries out the recited functionality or hardware that is programmed to perform the recited functionality. The hardware may be hardware disclosed herein or other known hardware that is programmed or configured to carry out the recited functionality. When the hardware is a processor that may be considered a type of circuitry, the circuitry, means, or units are a combination of hardware and software, and the software is used to configure the hardware and/or processor.

Aspects

It will be appreciated by those skilled in the art that the exemplary embodiments described above are specific examples of the following aspects.

(Item 1)

A robotic surgical system comprising:

a surgical apparatus including a first robot arm having an endoscope attached to a distal end thereof, and a second robot arm having a predetermined surgical instrument other than the endoscope attached to a distal end thereof;
an operation apparatus including an operation unit to receive an operation for the predetermined surgical instrument or the endoscope; and
a control device configured or programmed to perform a control to move the predetermined surgical instrument or the endoscope based on a received operation; wherein
the operation apparatus includes:

a display to display an image captured by the endoscope and rotate so as to be tilted with respect to a horizontal plane; and
a tilt detection sensor to detect a tilt of the display with respect to the horizontal plane; and

the control device is configured or programmed to correct translational movement of the predetermined surgical instrument based on the tilt detected by the tilt detection sensor.

(Item 2)

The robotic surgical system according to item 1, wherein the control device is configured or programmed to correct a direction of the translational movement of the predetermined surgical instrument based on a posture term that represents a posture of the endoscope based on a coordinate system of the endoscope and a correction term that corrects the posture term based on the tilt detected by the tilt detection sensor when a translational movement operation for the predetermined surgical instrument is received.

(Item 3)

The robotic surgical system according to item 2, wherein the control device is configured or programmed to correct a translation vector of the predetermined surgical instrument by multiplying a translation vector of the predetermined surgical instrument based on a coordinate system of the operation unit by a posture matrix of the endoscope based on the coordinate system of the endoscope and a posture correction matrix based on the tilt detected by the tilt detection sensor when the translational movement operation for the predetermined surgical instrument is received.

(Item 4)

The robotic surgical system according to any one of items 1 to 3, wherein

the operation apparatus includes a support arm to support the display; and
the support arm includes:

a plurality of links;
a plurality of joints to connect the plurality of links to each other; and
a brake to fix at least one of the plurality of joints such that the at least one of the plurality of joints does not rotate.

(Item 5)

The robotic surgical system according to any one of items 1 to 4, wherein

the operation apparatus includes a support arm to support the display; and
the support arm includes:

a plurality of links;
a joint to connect the plurality of links to each other; and
a spring to lift at least one of the plurality of links.

(Item 6)

The robotic surgical system according to any one of items 1 to 5, wherein the control device is configured or programmed to correct the translational movement of the predetermined surgical instrument based on the tilt detected by the tilt detection sensor during surgery in which the operation for the predetermined surgical instrument is received by the operation apparatus.

(Item 7)

The robotic surgical system according to any one of items 1 to 6, wherein

the tilt detection sensor is operable to detect a rotation angle of the display about a first axis along the horizontal plane; and
the control device is configured or programmed to correct the translational movement of the predetermined surgical instrument based on the rotation angle about the first axis detected by the tilt detection sensor.

(Item 8)

The robotic surgical system according to any one of items 1 to 6, wherein

the tilt detection sensor is operable to detect rotation angles of the display about a first axis along the horizontal plane, a second axis along the horizontal plane and perpendicular to the first axis, and a third axis along a vertical direction; and
the control device is configured or programmed to correct the translational movement of the predetermined surgical instrument based on the rotation angles about the first axis, the second axis, and the third axis detected by the tilt detection sensor.

(Item 9)

The robotic surgical system according to any one of items 1 to 8, wherein

the operation apparatus includes a holder to hold the display;
the display is operable to rotate about a first axis along the horizontal plane with respect to the holder; and
the tilt detection sensor includes an angle sensor to detect a rotation angle of the display about the first axis.

(Item 10)

The robotic surgical system according to any one of items 1 to 8, wherein the tilt detection sensor includes an acceleration sensor to detect at least a tilt of the display with respect to the horizontal plane.

(Item 11)

The robotic surgical system according to any one of items 1 to 10, wherein the operation apparatus further includes a switch to switch between a state in which a change in a tilt angle of the display with respect to the horizontal plane is permitted and a state in which the change in the tilt angle is not permitted.

(Item 12)

A control method for a robotic surgical system, the robotic surgical system comprising a surgical apparatus including a first robot arm having an endoscope attached to a distal end thereof and a second robot arm having a predetermined surgical instrument other than the endoscope attached to a distal end thereof, an operation apparatus including an operation unit to receive an operation for the predetermined surgical instrument or the endoscope, and a control device configured or programmed to perform a control to move the predetermined surgical instrument or the endoscope based on a received operation, the control method comprising:

> detecting, using a tilt detection sensor, a tilt with respect to a horizontal plane of a display operable to display an image captured by the endoscope, the display being operable to rotate so as to be tilted with respect to the horizontal plane; and
> correcting translational movement of the predetermined surgical instrument based on a detected tilt.

**Claims**

1. A robotic surgical system comprising:

> a surgical apparatus including a first robot arm having an endoscope attached to a distal end thereof, and a second robot arm having a predetermined surgical instrument other than the endoscope attached to a distal end thereof;
> an operation apparatus including an operation unit to receive an operation for the predetermined surgical instrument or the endoscope; and
> a control device configured or programmed to perform a control to move the predetermined surgical instrument or the endoscope based on a received operation; wherein
> the operation apparatus includes:
>
>> a display to display an image captured by the endoscope and rotate so as to be tilted with respect to a horizontal plane; and
>> a tilt detection sensor to detect a tilt of the display with respect to the horizontal plane; and
>
> the control device is configured or programmed to correct translational movement of the predetermined surgical instrument based on the tilt detected by the tilt detection sensor.

2. The robotic surgical system according to claim **1,**

wherein the control device is configured or programmed to correct a direction of the translational movement of the predetermined surgical instrument based on a posture term that represents a posture of the endoscope based on a coordinate system of the endoscope and a correction term that corrects the posture term based on the tilt detected by the tilt detection sensor when a translational movement operation for the predetermined surgical instrument is received.

3. The robotic surgical system according to claim **2,** wherein the control device is configured or programmed to correct a translation vector of the predetermined surgical instrument by multiplying a translation vector of the predetermined surgical instrument based on a coordinate system of the operation unit by a posture matrix of the endoscope based on the coordinate system of the endoscope and a posture correction matrix based on the tilt detected by the tilt detection sensor when the translational movement operation for the predetermined surgical instrument is received.

4. The robotic surgical system according to claim 1, wherein

> the operation apparatus includes a support arm to support the display; and
> the support arm includes:
>
>> a plurality of links;
>> a plurality of joints to connect the plurality of links to each other; and
>> a brake to fix at least one of the plurality of joints such that the at least one of the plurality of joints does not rotate.

5. The robotic surgical system according to claim 1, wherein

> the operation apparatus includes a support arm to support the display; and
> the support arm includes:
>
>> a plurality of links;
>> a joint to connect the plurality of links to each other; and
>> a spring to lift at least one of the plurality of links.

6. The robotic surgical system according to claim 1, wherein the control device is configured or programmed to correct the translational movement of the predetermined surgical instrument based on the tilt detected by the tilt detection sensor during surgery in which the operation for the predetermined surgical instrument is received by the operation apparatus.

7. The robotic surgical system according to claim 1, wherein

the tilt detection sensor is operable to detect a rotation angle of the display about a first axis along the horizontal plane; and
the control device is configured or programmed to correct the translational movement of the predetermined surgical instrument based on the rotation angle about the first axis detected by the tilt detection sensor.

8. The robotic surgical system according to claim 1, wherein

the tilt detection sensor is operable to detect rotation angles of the display about a first axis along the horizontal plane, a second axis along the horizontal plane and perpendicular to the first axis, and a third axis along a vertical direction; and
the control device is configured or programmed to correct the translational movement of the predetermined surgical instrument based on the rotation angles about the first axis, the second axis, and the third axis detected by the tilt detection sensor.

9. The robotic surgical system according to claim 1, wherein

the operation apparatus includes a holder to hold the display;
the display is operable to rotate about a first axis along the horizontal plane with respect to the holder; and
the tilt detection sensor includes an angle sensor to detect a rotation angle of the display about the first axis.

10. The robotic surgical system according to claim 1, wherein the tilt detection sensor includes an acceleration sensor to detect at least a tilt of the display with respect to the horizontal plane.

11. The robotic surgical system according to claim 1, wherein the operation apparatus further includes a switch to switch between a state in which a change in a tilt angle of the display with respect to the horizontal plane is permitted and a state in which the change in the tilt angle is not permitted.

12. A control method for a robotic surgical system, the robotic surgical system comprising a surgical apparatus including a first robot arm having an endoscope attached to a distal end thereof and a second robot arm having a predetermined surgical instrument other than the endoscope attached to a distal end thereof, an operation apparatus including an operation unit to receive an operation for the predetermined surgical instrument or the endoscope, and a control device configured or programmed to perform a control to move the predetermined surgical instrument or the endoscope based on a received operation, the control method comprising:

detecting, using a tilt detection sensor, a tilt with respect to a horizontal plane of a display operable to display an image captured by the endoscope, the display being operable to rotate so as to be tilted with respect to the horizontal plane; and
correcting translational movement of the predetermined surgical instrument based on a detected tilt.

*FIG.1*

100

*FIG.2*

*FIG.3*

FIG.4

*FIG.5*

*FIG.6*

FIG.7

*FIG.8*

*FIG.9*

*FIG.10*

*FIG.11*

*FIG.12*

*FIG.13*

*FIG.14*

*FIG.15*

FIG.16

HC COORDINATE SYSTEM

FIG.17

ENDOSCOPE COORDINATE SYSTEM

EP 4 516 255 A1

FIG.18

## FIG.19

```
              ( START )
                  │
                  ▼
┌────────────────────────────────────────────┐
│ DETECT TILT OF MONITOR USING ANGLE SENSOR   │── S1
└────────────────────────────────────────────┘
                  │
                  ▼
┌────────────────────────────────────────────┐
│ RECEIVE OPERATION FOR SURGICAL INSTRUMENT   │── S2
│           THROUGH OPERATION UNIT            │
└────────────────────────────────────────────┘
                  │
                  ▼
┌────────────────────────────────────────────┐
│ CORRECT TRANSLATION VECTOR OF SURGICAL      │── S3
│   INSTRUMENT BASED ON DETECTED TILT         │
└────────────────────────────────────────────┘
                  │
                  ▼
┌────────────────────────────────────────────┐
│ MOVE SURGICAL INSTRUMENT BASED ON           │── S4
│   CORRECTED TRANSLATION VECTOR              │
└────────────────────────────────────────────┘
                  │
                  ▼
              (  END  )
```

## FIG.20

## INTERNATIONAL SEARCH REPORT

| | International application No. |
|---|---|
| | **PCT/JP2023/023580** |

### A. CLASSIFICATION OF SUBJECT MATTER

*A61B 34/35*(2016.01)i; *B25J 3/00*(2006.01)i
FI: A61B34/35; B25J3/00

According to International Patent Classification (IPC) or to both national classification and IPC

### B. FIELDS SEARCHED

Minimum documentation searched (classification system followed by classification symbols)

A61B34/35; B25J3/00; G06F3/01

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

### C. DOCUMENTS CONSIDERED TO BE RELEVANT

| Category* | Citation of document, with indication, where appropriate, of the relevant passages | Relevant to claim No. |
|---|---|---|
| A | JP 2018-202032 A (MEDICAROID CORP.) 27 December 2018 (2018-12-27)<br>entire text, all drawings | 1-12 |
| A | JP 2020-162916 A (SONY CORP.) 08 October 2020 (2020-10-08)<br>entire text, all drawings | 1-12 |
| A | JP 2011-237987 A (OLYMPUS CORP.) 24 November 2011 (2011-11-24)<br>entire text, all drawings | 1-12 |
| A | JP 2017-54201 A (SONY INTERACTIVE ENTERTAINMENT LLC) 16 March 2017<br>(2017-03-16)<br>entire text, all drawings | 1-12 |

☐ Further documents are listed in the continuation of Box C.    ☑ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| **25 July 2023** | **01 August 2023** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (January 2015)

**INTERNATIONAL SEARCH REPORT**
Information on patent family members

International application No.

**PCT/JP2023/023580**

| Patent document cited in search report | | | Publication date (day/month/year) | Patent family member(s) | | | Publication date (day/month/year) |
|---|---|---|---|---|---|---|---|
| JP | 2018-202032 | A | 27 December 2018 | US entire text, all drawings CN | 2018/0353246 109009447 | A1 A | |
| JP | 2020-162916 | A | 08 October 2020 | US entire text, all drawings CN | 2022/0060678 113613847 | A1 A | |
| JP | 2011-237987 | A | 24 November 2011 | US entire text, all drawings CN | 2013/0063580 102869310 | A1 A | |
| JP | 2017-54201 | A | 16 March 2017 | US entire text, all drawings | 2019/0219824 | A1 | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20120283876